# EUROPEAN PATENT SPECIFICATION

(11) **EP 4 375 358 B1**
(45) Date of publication and mention of the grant of the patent: **31.12.2025**
(21) Application number: 22845907.9
(22) Date of filing: 19.07.2022
(51) Int. Cl.: A01N 1/146, A01N 1/147, C12M 1/00, C12M 1/12

(54) **BIOLOGICAL CELL PROCESSING WORK PLATE AND BIOLOGICAL CELL PRE-FREEZING PROCESSING WORK KIT**
ARBEITSPLATTE ZUR VERARBEITUNG BIOLOGISCHER ZELLEN UND ARBEITSKIT ZUR VERARBEITUNG ZUM VORGEFRIEREN BIOLOGISCHER ZELLEN
PLATEAU DE TRAVAIL POUR LE TRAITEMENT DE CELLULES BIOLOGIQUES ET KIT DE TRAVAIL POUR LE TRAITEMENT DE PRÉCONGÉLATION DE CELLULES BIOLOGIQUES

(30) Priority: 21.07.2021 JP 2021121041
(43) Date of publication of application: 29.05.2024
(73) Proprietor: Kitazato Corporation, Fuji-shi, Shizuoka 416-0932 (JP)
(72) Inventor: INOUE Futoshi, Fuji-shi, Shizuoka 416-0932 (JP)
(74) Representative: Casalonga
(86) International application number: PCT/JP2022/028017
(87) International publication number: WO 2023/002974

(56) References cited:
- EP-B1- 1 516 532
- WO-A1-2016/190322
- WO-A1-2019/017464
- WO-A1-2020/013172
- WO-A1-2020/013174
- WO-A1-2021/025017
- JP-A- 2019 154 285
- JP-U- 3 226 911
- JP-U- 3 226 911
- JP-U- 3 230 875
- US-A1- 2007 074 990

## Description

### Technical Field

The invention relates to a plate for biological cell processing operation for use in various treatments of biological cells, e.g. pre-treatment of biological cells such as mammalian eggs, embryos and other eggs, sperm, hematopoietic stem cells, pluripotent stem cells and other stem cells for cryopreservation, thawing of cryopreserved biological cells operation and kit for pre-treatment operation of biological cell cryopreservation. In particular, the present invention relates to a plate for biological cell processing operation.

### Background Art

Cryopreservation of mammalian embryos enables preservation of genetic resources of specific breeds and species. It is also effective for maintaining animal species that stand on the verge of extinction. In addition, it is also useful for treatment of human infertility.

As a method for cryopreserving mammalian embryos, JP 2000-189155A (Patent Document 1) has proposed a method in which mammalian embryos or ova are caused to adhere to the inner surface of a cryopreservation container, such as a frozen straw, frozen vial, or frozen tube that has been sterilized, using the least amount of a vitrification solution sufficient to encapsulate these embryos or ova, and thereafter, the cryopreservation container is hermetically sealed and then rapidly cooled by contact with liquid nitrogen.

The inventors of the present invention have proposed Patent Document 2 (JP 2002-315573A, WO 02/085110 A1). Patent Document 2 discloses an egg cryopreservation tool 1 including a main body 2 made of a cold-resistant material, an egg adhesion holding strip 3 attached to one end of the main body 2 and made of a material that is flexible, transparent, and resistant to liquid nitrogen, and a cylindrical member 4 detachably attached to the main body 2 in a manner capable of covering the egg adhesion holding strip 3, sealed at one end, and made of a cold-resistant material.

JP 2017-118884A (Patent Document 3) has proposed a cell treatment container for use in cell treatments using a treatment solution, including a treatment of embryos immediately before transfer.

The applicant has also proposed a plate for pre-treatment operation of biological cell cryopreservation in WO 2021/025017 (Patent Document 4).

In addition, Japanese Registered Utility Model No. 3226911 (Patent Document 5) discloses a cell manipulation apparatus 1 for use in manipulating cells. The above-mentioned cell manipulation apparatus 1 has a body part 2 having a first well 8 filled with a first solution, and a sealing member 3 provided on the upper surface of the body part 2 in a peelable state. With the first solution filled into the first well 8, the sealing member 3 is adhered to the periphery 20A of the opening 20 of the first well 8 and the first well 8 is sealed by the sealing member 3.

### Citation List

### Patent Documents

Patent Document 1: JP 2000-189155A
Patent Document 2: JP 2002-315573A (WO 02/085110 A1)
Patent Document 3: JP 2017-118884A (WO 2016/190322 A1)
Patent Document 4: WO 2021/025017
Patent Document 5: Japanese Registered Utility Model No. 3226911

### Summary of Invention

### Technical Problem

The above-mentioned patent documents 1 to 4 are simply working plates, and the biological cell processing solution has to be prepared by the operator. It is desired that the biological cell processing work can be prepared quickly. The one in Patent Document 5 does not require the preparation of liquid, but the sealing material is not easy to peel off.

It is an object of the present invention to provide a plate for biological cell processing operation which is easily prepared for biological cell processing without preparing a biological cell processing solution and which allows a biological cell processing operation to be started quickly, and a kit for pre-treatment operation of biological cell cryopreservation.

### Solution to Problem

The invention provides as means for achieving the above-described object the following: a plate according to independent claim 1 and a kit according to independent claim 10. The dependent claims relate to advantageous embodiments.

### Brief Description of Drawings

Fig. 1 is a plan view of an example of a kit for pre-treatment operation of biological cell cryopreservation.
Fig. 2 is a cross-sectional view taken along line A-A of a plate for biological cell processing operation of the invention (a first step plate for the biological cell processing operation) used in the kit for pre-treatment operation of biological cell cryopreservation of Fig. 1.
Fig. 3 is a cross-sectional view of the plate for biological cell processing operation (the first step plate for the biological cell processing operation) shown in Fig. 2 with a lid removed.
Fig. 4 is a front view of a plate for biological cell processing operation of the invention (a second step plate for the biological cell processing operation) used in the kit for pre-treatment operation of biological cell cryopreservation of Fig. 1.
Fig. 5 is a cross-sectional view taken along line B-B of the plate for biological cell processing operation of the invention (the second step plate for the biological cell processing operation) shown in Fig. 4.
Fig. 6 is a plan view of a plate body of the plate for the biological cell processing operation of the invention (the second step plate for the biological cell processing operation) shown in Fig. 4.
Fig. 7 is a front view of a plate body of the plate for the biological cell processing operation of the invention (the second step plate for the biological cell processing operation) shown in Fig. 4.
Fig. 8 is a bottom view of a lid of the plate for the biological cell processing operation of the invention (the second step plate for the biological cell processing operation) shown in Fig. 4.
Fig. 9 is a right side view of the lid of the plate for the biological cell processing operation of the invention (the second step plate for the biological cell processing operation) shown in Fig. 4.
Fig. 10 is a plan view of an example of a sealing sheet used in the kit for pre-treatment operation of biological cell cryopreservation and the plate for biological cell processing operation of the invention.
Fig. 11 is a plan view of the plate for biological cell processing operation (the second step plate for the biological cell processing operation) shown in Fig. 4 with a lid removed.
Fig. 12 is a front view of the plate for biological cell processing operation (the second step plate for the biological cell processing operation) shown in Fig. 4 with a lid removed.
Fig. 13 is a cross-sectional view taken along line C-C of the plate for biological cell processing operation of the invention (the second step plate for the biological cell processing operation) shown in Fig. 12.
Fig. 14 is a plan view of another example of the plate for the biological cell processing operation of the invention (the second step plate for the biological cell processing operation).
Fig. 15 is a cross-sectional view taken along line D-D of the plate for biological cell processing operation shown in Fig. 14.
Fig. 16 is a plan view of the plate for biological cell processing operation shown in Fig. 14.
Fig. 17 is a cross-sectional view of another example of the plate for the biological cell processing operation of the invention (the second step plate for the biological cell processing operation).

### Description of Embodiments

The kit for pre-treatment operation of biological cell cryopreservation and the plate for biological cell processing operation of the invention are described using the examples shown in the drawings.

The plate for biological cell processing operation of the invention 1a has a plate body 2a, the plate body 2a has a recess 8, the recess 8 is filled with biological cell processing solution 9 and an opening of the recess 8 is sealed by a sealing sheet 4 which is peelable.

The plate for biological cell processing operation 1a has a lid 3a covering the recess 8. The plate 1a has a base portion 5a and an annular wall portion 6a extending above the base portion 5a. The annular wall portion 6a has the recess 8 located on an inner side thereof. The base portion 5a has an upper surface portion 52a located between the annular wall portion 6a and the outer edge of base portion 5a.

The lid 3a, when fitted on the plate 1a, covers the annular wall 6a and the recess 8 and rests on the base portion 5a of the plate 1a. The sealing sheet 4 has a sheet body portion 41 sealing the opening of the recess 8 and a gripping portion for peeling 42 extending from the sheet body portion 41.

When the lid 3a covers the annular wall portion 6a and the recess 8 of the plate 1a and rests on the base portion 5a of the plate 1a, the gripping portion for peeling 42 passes between the annular wall 6a and the lid 3a, and is located on the upper surface portion 52a of the base portion 5a, and is pushed by the lower edge of the lid 3a, and is in close proximity to the upper surface of the upper surface portion 52a, and cannot be gripped. And when the lid 3a is removed from the plate, the gripping portion for peeling 42 can be gripped by raising up and away from the upper surface of the upper surface portion 52a.

The kit for pre-treatment operation of biological cell cryopreservation 10 comprises a first step plate for biological cell processing operation (plate for biological cell processing operation) 1a and second step plate for biological cell processing operation (plate for biological cell processing operation) 1b. The first step plate for biological cell processing operation 1a has a recess 8. The recess 8 is filled with the biological cell processing solution 9 for the first step. The opening of the recess 8 is sealed by the sealing sheet 4, which is peelable. The second step plate for biological cell processing operation 1b has a flat plate portion 20b and a first recess 21 and a second recess 22 in the flat plate portion 20b. The first recess 21 is filled with first biological cell processing solution 11 for the second step and the second recess 22 is filled with second biological cell processing solution 12 for the second step. The opening of the first recess 21 and the opening of the second recess 22 are sealed by the sealing sheets 4a, 4b, which are removable.

The plate for biological cell processing operation 1a, 1b in this example has a plate (plate body) 2a, 2b and a lid 3a, 3b encasing a flat plate portion including a recess of the plate (plate body) 2a, 2b. The plate (plate body) 2a, 2b has a base portion 5a, 5b and an annular wall portion 6a, 6b extending above the base portion 5a, 5b. Recesses 8, 21, 22, 23 are provided inside the annular wall portions 6a, 6b. The base portions 5a, 5b are provided with upper surface portions 52a, 52b located between the outer edges of the annular wall portions 6a, 6b and the base portions 5a, 5b. The lids 3a, 3b cover the annular wall portions 6a, 6b and the recesses 8, 21, 22, 23 and rest on the base portions 5a, 5b of the plate body 2a, 2b when placed on the plate body 2a, 2b. The sealing sheet 4 (4a, 4b, 4c) has a sheet body portion 41 sealing the openings of the recesses 8, 21, 22, 23 and a gripping portion for peeling 42 extending from the sheet body portion 41.

When the lids 3a, 3b cover the annular wall portions 6a, 6b and flat plate portions 20a, 20b of the plate body 2a, 2b and are placed on the base portions 5a, 5b of the plate body 2a, 2b, the portion for peeling 42 passes between the annular wall portions 6a, 6b and the lids 3a, 3b and is located on the upper surface portions 52a, 52b of the base portion 5a, 5b and is pressed at the lower edge 32b of the lid 3a, 3b, close to the upper surface of the upper surface portions 52a, 52b and is ungraspable. When the lids 3a, 3b are removed from the plate body 2a, 2b, the gripping portion for peeling 42 is raised up and away from the upper surface of the upper surface portions 52a, 52b and can be gripped.

The kit for pre-treatment operation of biological cell cryopreservation 10 comprises a first step plate for biological cell processing operation 1a, which is a plate for biological cell processing operation, a lid 3a, which is removably attached (fitted) to the first step plate for biological cell processing operation 1a, a second step plate for biological cell processing operation 1b, which is a plate for biological cell processing operation, and a lid 3b is removably attached (fitted) to the second step plate for biological cell processing operation 1b.

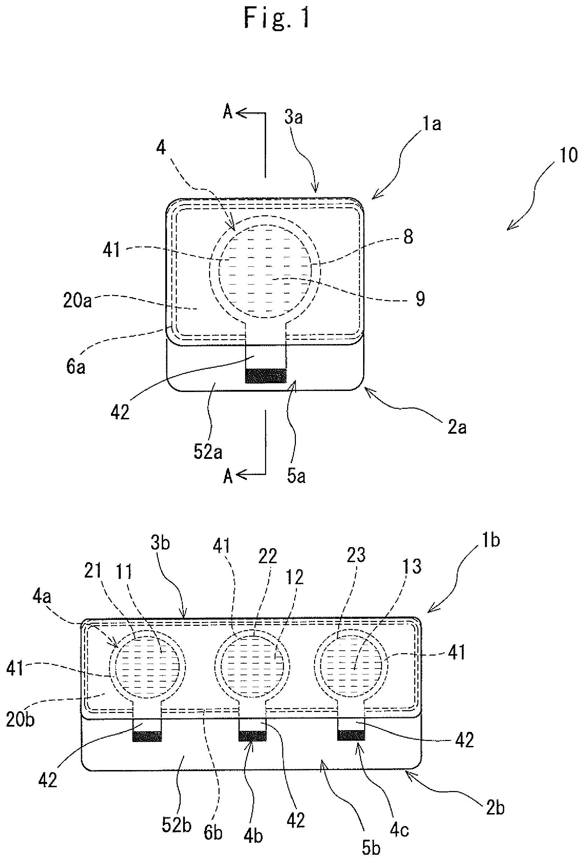

The first step plate for biological cell processing operation 1a, which is a plate for biological cell processing operation, is provided with a plate body 2a. The plate body 2a is provided with a base portion 5a and an annular wall portion 6a extending upwardly from the base portion 5a, and a flat plate portion 20a inside the annular wall portion 6a, as shown in Figs. 1 to 3. In this example of the plate body 2a, the flat plate portion 20a is located inside the annular wall portion 6a and at the upper end of the annular wall portion 6a.

The base portion 5a is provided with an upper surface portion 52a located between the annular wall portion 6a and the outer edge of the base portion 5a. The lid 3a covers the annular wall portion 6a and the flat plate portion 20a when mounted on the plate body 2a and is placed on the base portion 5a (specifically, on the upper surface portion 52a) of the plate body 2a. The base portion 5a has an annular leg portion 51a and an upper surface portion 52a at its upper end.

The upper surface portion 52a is provided with a pressable portion that can be pressed by a finger during a peeling operation of the sealing sheet 4 using the gripping portion for peeling 42. Specifically, the upper surface portion 52a is provided with a flat plate portion extending to the front side at the part that is in front of the recess 8 during the peeling operation of the sealing sheet 4. This portion can be pressed by a finger and can be used as a pressable portion (pressing area). Thus, for example, the sealing sheet 4 can be peeled off by pressing the upper surface portion 52a with the finger of the left hand, holding the plate, then grasping the gripping portion for peeling 42 and pulling it up, without leaking the liquid filled in the recesses.

The height of the base 5a (annular leg 51a) is preferably 2 to 7 mm, especially 3 to 5 mm, and the height of the annular wall 6a is preferably 1 to 5 mm, especially 1.5 to 4 mm.

In this example of the plate body 2a, the flat plate portion 20a is provided with a recess 8. The diameter of the opening of the recess 8 is preferably 15-45 mm, in particular 20-40 mm. The depth of the recess 8 is preferably 5-20 mm, in particular 7-15 mm. In this example, the recess 8 is a partially spherical recess.

The recess 8 is filled with biological cell processing solution 9. As biological cell processing solution 9, a biological cell processing solution suitable for the purpose of cell processing is used. If plate 1a in this example is a first step plate for biological cell processing operation of the kit for pre-treatment operation of biological cell cryopreservation 10, a treatment solution that does not contain cell membrane-permeable cryoprotectant (treatment solution free of cell membrane-permeable cryoprotectant)9 is filled with the recess 8.

As BS (treatment solution free of cell membrane-permeable cryoprotectant), those conventionally used are suitably used. As the treatment solution free of cell membrane-permeable cryoprotectant, it is suitable to use, for example, a basal cell culture medium (e.g., a cell culture medium containing HEPES [4-(2-HydroxyEthyl)-1-PiperazineEthaneSulfonic acid]) that does not contain a cell membrane-permeable cryoprotectant and contains compounds considered necessary, such as, for example: one type or two or more types of cell membrane-impermeable cryoprotectants selected from sucrose, trehalose, Percoll, polyethylene glycol, polyvinyl pyrrolidone, bovine serum albumin, Ficoll, and at least one type of water-soluble cellulose selected from the group consisting of hydroxypropyl cellulose, hydroxypropyl methylcellulose, and hydroxyethyl methylcellulose, and an antibiotic such as gentamicin.

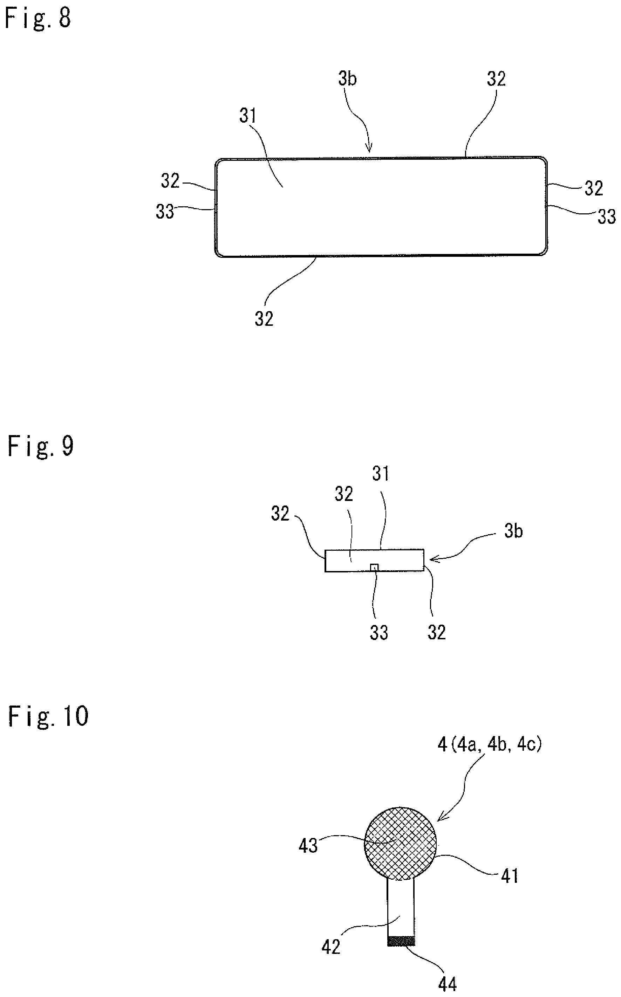

The opening of the recess 8 is sealed by the sealing sheet 4 which is peelable, and the sealing sheet 4 prevents the liquid filled in the recess 8 from flowing out. In this example, the sealing sheet 4 has a sheet body portion 41 sealing the opening of the recess 8 and a gripping portion for peeling 42 extending from the sheet body portion 41, as shown in FIG. 10. An adhering substance 43, such as an adhesive substance or a pressure-sensitive adhesive, is provided on the lower surface of the sheet body portion 41. The adhering substance may be provided only on the periphery of the lower surface of the sheet body portion 41.

The sheet body portion 41 is larger than the opening of the recess 8. The outer peripheral portion of the opening of the recess 8, which is part of the flat plate portion, is flat. The outer edge side portion of the sheet body portion 41 of the sealing sheet 4 is peelably attached to the flat peripheral portion. The width of the sealing sheet (sheet body portion 41) attached to the flat plate portion is preferably 0.7 mm or more. In particular, it is preferably 1 mm or more.

The free end of the gripping portion for peeling 42 is provided with an end marker 44. The sealing sheet 4, especially the sheet body portion 41, is preferably a gas impermeable sheet. If the sheet body portion 41 is such, denaturation and concentration changes of the biological cell processing solution 9 filled in the recess 8 can be suppressed. The sealing sheet 4 includes a gripping portion for peeling 42 and is a flexible sheet.

The sealing sheet 4 should have a certain degree of strength, flexibility, and gas impermeability. The sealing sheet 4 can be, for example, ethylene-vinyl acetate copolymer, polyvinyl alcohol, polyvinylidene chloride, polyamide, polyester, polyacrylonitrile, vinylidene chloride-acrylonitrile copolymer, acrylonitrile methyl methacrylate-butadiene copolymer, nylon 6, biaxially oriented nylon, biaxially oriented polyethylene terephthalate, biaxially oriented polypropylene, high density polyethylene, ionomer resin, a metal vapor deposited film alone or a combination of metal vapor deposited film and the above polymers is suitable. The thickness of the sealing sheet 4 is preferably 5 to 300 µm, especially 25 to 200 µm.

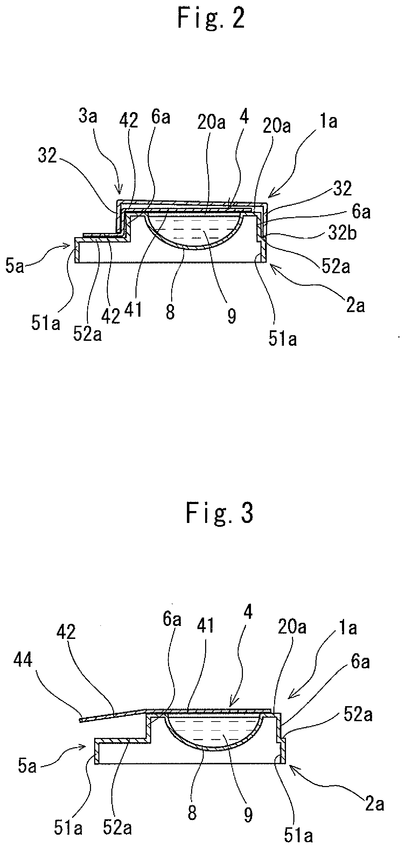

In this example plate for biological cell processing operation 1a, when the lid 3a is placed (fitted) on the plate (plate body) 2a (Figs. 1 and 2), the gripping portion for peeling 42 of the sealing sheet 4 is bent, passes through the annular wall portion (outer surface) 6a and the lid (inner surface) 3a, and is located on the upper surface portion 52a of the base portion 5a. The peeling grip portion 42 is located on the upper surface portion 52a of the base portion 5a and is pressed by the lower edge 32b of the lid 3a. The peeling gripping portion 42 is pressed by the lower edge 32b of the lid 3a and is close to the upper surface of the upper surface portion 52a. The gripping portion for peeling 42 so that it cannot be grasped.

Furthermore, in this example of the plate for biological cell processing operation 1a, when the lid 3a is removed from the plate body 2a, it is in the state shown in Figure 3. The gripping portion for peeling 42 rises up and leaves the upper surface of the upper surface portion 52a. The gripping section 42 for peeling can be gripped. In particular, in this example of the plate for biological cell processing operation 1a, the free end of the gripping portion for peeling 42 extends beyond the edge of the plate (plate body) 2a. The free end of the gripping portion for peeling 42 is easier to grasp.

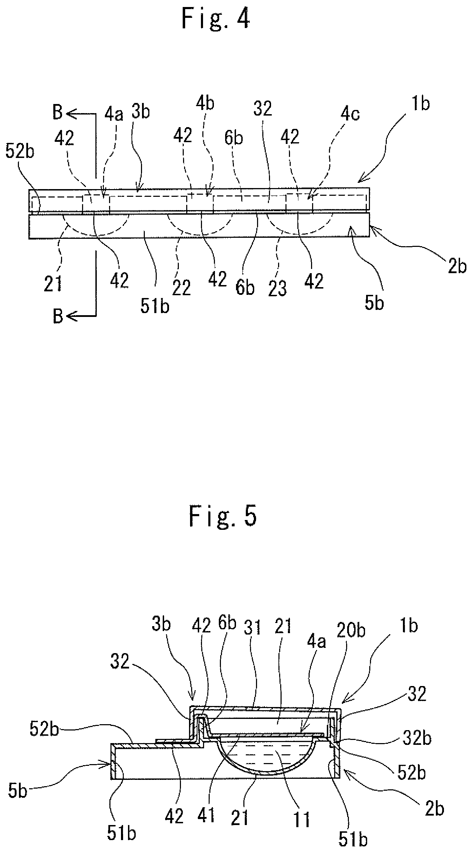

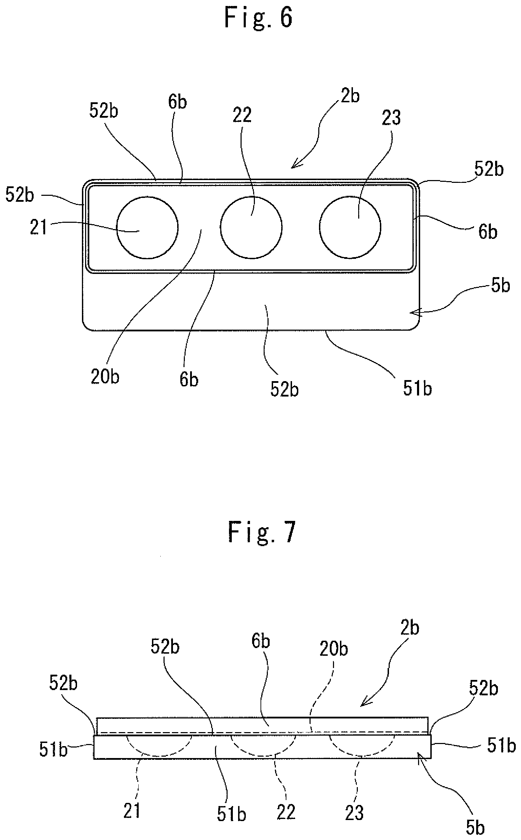

The second step plate for biological cell processing operation 1b, which is a plate for biological cell processing operation, has a plate body 2b and a lid 3b attached (placed) to the plate body 2b. The plate body 2b of the second step plate for biological cell processing operation has a base portion 5b and an annular wall portion 6b extending upward from the base portion 5b, as shown in Figs. 1, 4 through 7. The plate body 2b has a flat plate portion 20b inside the annular wall portion 6b. In this example of the plate body 2b, the flat plate portion 20b is located inside and below the upper edge of the annular wall portion 6b. Therefore, the annular wall portion 6b protrudes upward from the flat plate portion 20b.

The base portion 5b has an upper surface portion 52b located between the annular wall portion 6b and the outer edge of the base portion 5b. The lid 3b, when placed on the plate body 2b, encloses the annular wall portion 6b and the flat plate portion 20b and is placed on the base portion 5b (specifically, on the upper surface portion 52b) of the plate body 2b. The base portion 5b has an annular leg portion 51b and an upper surface portion 52b at its upper end.

The height of the base portion 5b (annular leg portion 51b) is preferably 1 to 5 mm, especially 1.5 to 4 mm. The height of the annular wall portion 6b is preferably 1 to 5 mm, especially 1.5 to 4 mm.

The plate body 2b of this example, the flat plate portion 20b, has not only a first recess 21 but also a second recess 22 and a third recess 23. The number of recesses may be two, four or more. The diameter of the openings of the recesses 21, 22, 23 is preferably 5 to 20 mm, specially 7 to 15 mm. The depth of the recesses 21, 22, 23 is preferably 3 to 15 mm, especially 4 to 10 mm. In this example, the recesses 21, 22, 23 are hemispherical recesses.

In the plate 1b of this example, the first recess 21, second recess 22, and third recess 23 are filled with biological cell processing solutions 11, 12, 13. When the recesses have a first recess and a second recess, the biological cell processing solution filled in the first recess and the biological cell processing solution filled in the second recess should be different. Furthermore, one biological cell processing solution preferably have a color that is distinguishable from the other biological cell processing solution.

When the recesses have a first, second and third recess, at least one of the three biological cell processing solutions 11, 12, 13 filled in the first, second and third recesses 21, 22 and 23 should be different from the other biological cell processing solution. In addition, the different biological cell processing solution and the remaining two biological cell processing solutions preferably have a color which allows them to be distinguished from each other.

An effective way of giving the biological cell processing solution a distinctive color is to add a coloring agent to one of the two solutions. Any coloring agent that is normally allowed to be added to medicines can be used as a coloring agent. Examples of the coloring agent include Red No.2 (Amaranth), Red No.3 (Erythrosine), Red No.102 (New Coxin), Yellow No.4 (Tartrazine), Yellow No.5 (Sunset Yellow FCF), Green No.3 (Fast Green FCF), Blue No.1 (Brilliant Blue FCF), Blue No.2 (Indigo Carmine) and their aluminum lake.

As biological cell processing solutions 11, 12, 13, biological cell processing solutions adapted to the purpose of cell processing are used. If plate 1b in this example is the second step plate for biological cell processing in the kit for pre-treatment operation of biological cell cryopreservation operation 10, the first recess 21 is filled with a processing solution containing a cell membrane-permeable cryoprotectant. Specifically, the first recess 21 is filled with a low-concentration processing solution (ES: Equilibration Solution) containing a low concentration of cell membrane-permeable cryoprotectant.

As low concentration treatment solution (ES) that contains a cell membrane-permeable cryoprotectant at a low concentration, it is suitable to use, for example, a basal cell culture medium (e.g., a cell culture medium containing HEPES [4-(2-HydroxyEthyl)-1-PiperazineEthaneSulfonic acid]) that contains a cell membrane-permeable cryoprotectant such as glycerol, propylene glycol, dimethylsulfoxide (DMSO), ethylene glycol, or butanediol at a low concentration and also contains a cell membrane-impermeable cryoprotectant such as sucrose, trehalose, Percoll, polyethylene glycol, polyvinyl pyrrolidone, bovine serum albumin, Ficoll, hydroxypropyl cellulose, hydroxypropyl methylcellulose, or hydroxyethyl methylcellulose. Antibiotics such as gentamicin may also be included in this ES.

The content of the cell membrane-permeable cryoprotectant in this ES is preferably 20% to 70% and particularly preferably 30% to 60% of the content of a cell membrane-permeable cryoprotectant in "VS" (high-concentration treatment solution that contains a cell membrane-permeable cryoprotectant at a high concentration) to be described below.

If the plate 1b in this example is the kit for pre-treatment operation of biological cell cryopreservation 10 second step plate for biological cell processing operation, the second recess 22 and the third recess 23 are filled with a processing solution containing a cell membrane-permeable cryoprotectant. Specifically, a high concentration treatment solution (VS: Vitrification Solution) containing a high concentration of cell membrane-permeable cryoprotectant is filled.

As the high-concentration treatment solution (VS) that contains a cell membrane-permeable cryoprotectant at a high concentration, it is suitable to use, for example, a basal cell culture medium (e.g., a cell culture medium containing HEPES [4-(2-HydroxyEthyl)-1-PiperazineEthaneSulfonic acid]) that contains a cell membrane-permeable cryoprotectant such as glycerol, propylene glycol, dimethylsulfoxide (DMSO), ethylene glycol, or butanediol at a high concentration and also contains a cell membrane-impermeable cryoprotectant such as sucrose, trehalose, Percoll, polyethylene glycol, polyvinyl pyrrolidone, bovine serum albumin, Ficoll, hydroxypropyl cellulose, hydroxypropyl methylcellulose, or hydroxyethyl methylcellulose.

This VS also may contain an antibiotic such as gentamicin. The content of the cell membrane-permeable cryoprotectant in this VS is preferably 1.5 to 3 times and particularly preferably 1.7 to 2.5 times the content of the cell membrane-permeable cryoprotectant in the above-described "ES" (low-concentration treatment solution that contains a cell membrane-permeable cryoprotectant at a low concentration).

The opening of the first recess 21 is sealed by the sealing sheet 4a, which is peelable, the opening of the second recess 22 is sealed by the sealing sheet 4b, which is peelable, and the opening of the third recess 23 is sealed by the sealing sheet 4c, which is peelable. The liquids filled in the respective recesses are not allowed to flow out.

In this example, the sealing sheets 4a, 4b and 4c shown in Fig. 10 and described above can also be suitably used.

The lid 3b of the second step plate for biological cell processing operation covers the entire annular wall portion 6b and flat plate portion 20b described above, as shown in Figures 1, 4, 5, 8 and 9. When the lid 3b covered the plate, most of the upper surface portion 52b of the base portion 5b is exposed. Therefore, the lid 3b does not obstruct work such as writing on the upper surface portion 52b. The lid 3b in this example also has a top plate 31 and an annular side wall 32 extending below the top plate 31. The annular side wall 32 is provided with slits 33 on the sides facing each other.

In this example plate for biological cell processing operation 1b, when the lid 3b is attached (placed) on the plate (plate body) 2b (Fig. 1, Fig. 4, Fig. 5), the gripping portion for peeling 42 of the sealing sheet 4a, 4b, 4c bends and extends upwards on the inner surface of the annular wall portion 6b, the gripping portion bends again and passes between the outer surface of the annular wall portion 6b and the lid (inner surface) 3b and is located on the upper surface portion 52b of the base portion 5b. The gripping portion for peeling is then pushed by the lower edge 32b of the lid 3b and is close to the upper surface of the upper surface portion 52b, making it ungraspable.

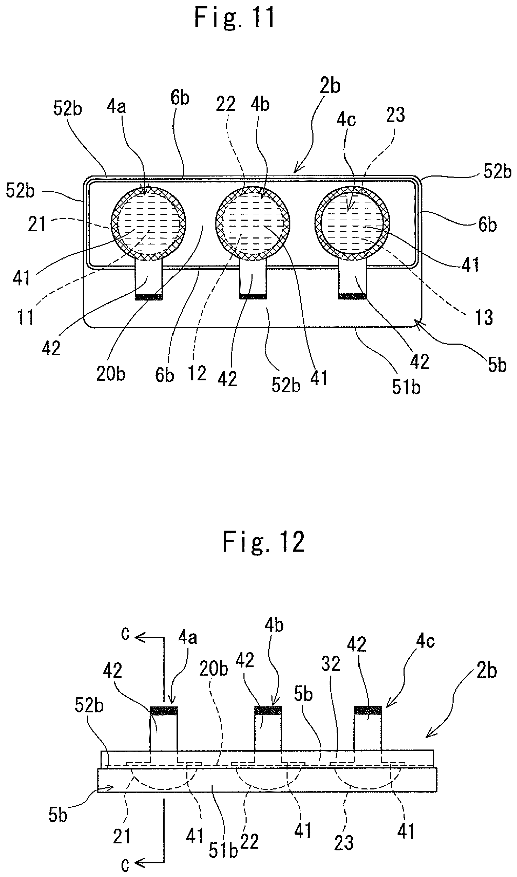

In this example plate for biological cell processing operation 1b, when the lid 3b is removed from the plate (plate body) 2b, the state is as shown in Figures 11, 12 and 13. The gripping portion for peeling 42 rises, leaves the upper surface portion 52b and protrudes obliquely from the upper edge of the annular wall portion 6b and can be gripped. In particular, in this example of the plate for biological cell processing operation 1b, the free end of the gripping portion for peeling 42 protrudes obliquely from the upper edge of the annular wall portion 6b of the plate body 2b, the peeling gripping portion 42 is easy to grip the same.

The plate for biological cell processing operation 1a, 1b and lids 3a, 3b are formed from synthetic resin material. The plate for biological cell processing operation and the lid should be highly transparent, and a transparent hard synthetic resin is suitable. Transparent hard synthetic resins include styrene resins such as polystyrene and SBS, polycarbonate, acrylonitrile resins, polyolefins such as polypropylene and polyethylene, polyvinyl chloride, polyester resins such as PMMA (polymethyl methacrylate), polyethylene terephthalate and polybutylene terephthalate and are preferred.

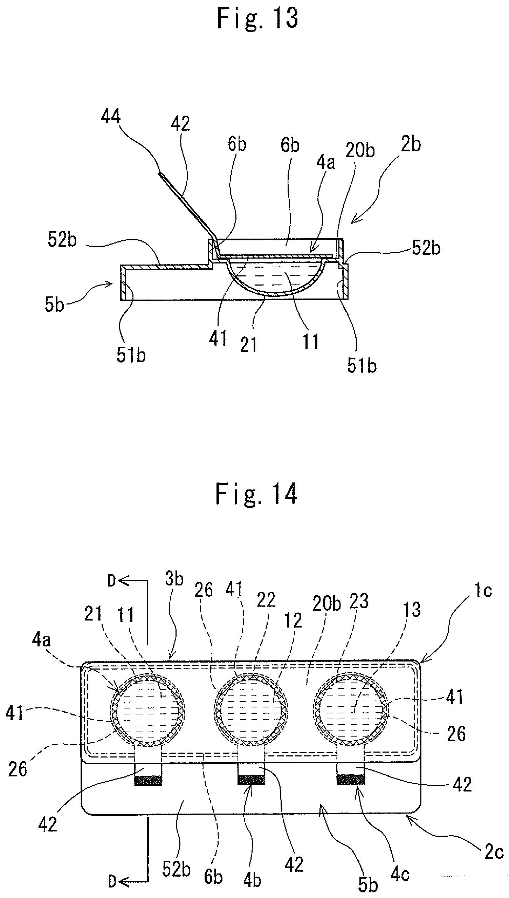

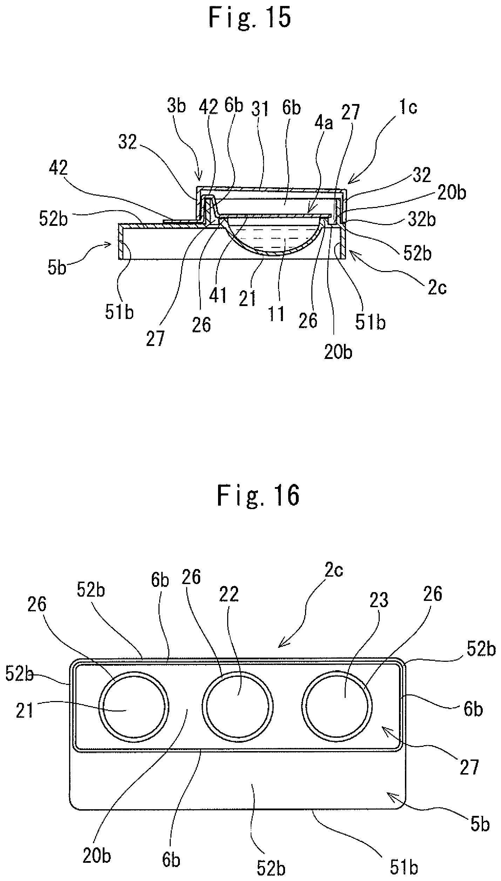

The plate for biological cell processing operation may be a plate for biological cell processing operation 1c, as shown in Figures 14 to 16. The basic configuration of the plate for biological cell processing operation 1c in this example is the same as that of the plate for biological cell processing operation 1b described above. The only difference is that the plate body 2c has annular ribs 26 at the periphery of the openings of the first recess 21, the second recess 22 and the third recess 23. And in this example, the plate body 2c has a working use liquid storage area 27 (waste liquid storage area) formed by the flat plate portion 20b, the annular wall portion 6b and the above three annular ribs 26 on the flat plate portion 20b. Furthermore, the sealing sheets 4a, 4b and 4c are attached to the upper surface of these annular ribs 26. For this reason, the upper surface of the annular rib 26 is preferably flat, as shown in the figure.

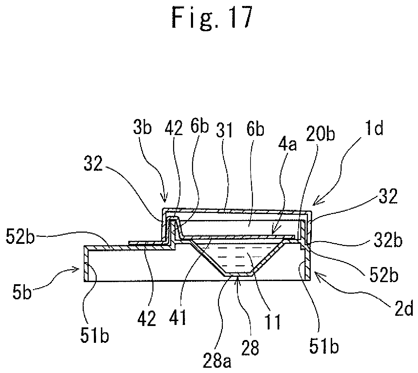

In all the examples described above, the shape of the recess is not limited to those described above. The shape of the recess may be in the form of an inverted cone with a flat base 28a, such as the recess 28 in the plate body 2d of the plate for biological cell processing operation 1d shown in Figure 17.

The plate for biological cell processing operation has a recess, a biological cell processing solution filled into the recess and the sealing sheet peelable at the opening of the recess. Furthermore, the plate for biological cell processing operation has a lid covering the recess, the plate having a base portion and an annular wall portion extending above the base portion. The recess is located inside the annular wall portion. The base portion has an upper surface portion located between the outer edge of the annular wall portion and the base portion.

The lid covers the annular wall portion and the recess when mounted on the plate and is placed on the base portion of the plate. The sealing sheet has a sheet body portion sealing the recess opening and a gripping portion for peeling extending from the sheet body portion. When the lid covers the annular wall portion and the recess of the plate and is placed on the base portion of the plate, the gripping portion for peeling passes between the annular wall portion and the lid and is located on the gripping portion for peeling passes between the annular wall portion and the lid and is located on the upper surface portion of the base portion. The gripping portion is pushed by the lower edge of the lid and is close to the upper surface of the upper surface portion, making it ungraspable. When the lid is removed from the plate, the gripping portion for peeling is raised up and away from the upper surface of the upper surface portion and can be gripped.

For this reason, unnecessary peeling of the sealing sheet is prevented because it is practically impossible to peel the sealing sheet when the lid is in place. By removing the lid, the gripping portion for peeling is raised up and away from the upper surface of the upper surface portion and can be grasped.
This makes it easier to recognize a peeling start portion and also makes it easier to peel off the sealing sheet. This allows the opening of the recess filled with biological cell processing solution to be opened quickly.

## Claims

1. A plate (1a) for biological cell processing operation having a recess (8), a biological cell processing solution filled in the recess (8) and a sealing sheet (4) peelably provided at the opening of the recess (8),
wherein the plate (1a) for the biological cell processing operation has a removable lid (3a) covering the recess (8),
the plate (1a) has a base portion (5a) and an annular wall portion (6a) extending upward from the base portion (5a), and the recess (8) is located inside the annular wall portion (6a),
the base portion (5a) has an upper surface portion (52a) located between the annular wall portion (6a) and an outer edge of the base portion (5a),
the lid (3a) is fitted on the plate (1a) and covers the annular wall portion (6a) and the recess (8) and rests on the base portion (5a) of the plate (1a),
the sealing sheet (4) has a sheet body portion (41) for sealing the opening of the recess (8),
wherein
a gripping portion (42) is provided for peeling that extends from the sheet body portion (41), and
the lid (3a) covers the annular wall portion (6a) and the recess (8) of the plate (1a) and rests on the base portion (5a) of the plate, the gripping portion (42) for peeling passes between the annular wall portion (6a) and the lid (3a), is located on the upper surface portion (52a) of the base portion (5a), and is pushed by the lower edge of the lid (3a), and is in close proximity to the upper surface of the upper surface portion (52a), and cannot be gripped, and when the lid (3a) is removed from the plate (1a), the gripping portion (42) for peeling can be gripped by raising up and away from the upper surface of the upper surface portion (52a).

2. The plate (1a) for biological cell processing operation according to claim 1, wherein the upper surface portion (52a) of the base portion (5a) of the plate has a pressable portion which can be pressed by a finger when a performing peeling work of the sealing sheet (4) by using the gripping portion (42) for peeling.

3. The plate (1a) for biological cell processing operation according to claim 1 or 2, wherein the plate (1a) has a flat plate portion (20b) provided inside the annular wall portion (6a) and the recess (8) is provided in the flat plate portion (20b).

4. The plate (1a) for biological cell processing operation according to claim 3, wherein the flat plate portion (20b) is located inside the annular wall portion (6a) and below a top edge of the annular wall portion (6a).

5. The plate (1a) for biological cell processing operation according to claim 4, wherein the plate (1a) has an annular rib provided at a periphery of the recess (8) and a liquid reservoir formed by the flat plate portion (20b), the annular rib and the annular wall portion (6a), and the sealing sheet (4) is peelably adhered to an upper surface of the annular rib.

6. The plate (1b) for biological cell processing operation according to any one of claims 1 to 5, wherein the plate (1b) has a first recess (21) and a second recess (22), the second recess (22) being filled with a biological cell processing solution, an opening of the second recess (22) being sealed by a sealing sheet (4b) which is peelable.

7. The plate (1b) for biological cell processing operation according to claim 6, wherein the biological cell processing solution filled in the first recess (21) and the biological cell processing solution filled in the second recess (22) are different, and wherein one biological cell processing solution has a color that is distinguishable from the other biological cell processing solution.

8. The plate (1b) for biological cell processing operation according to any one of claims 1 to 5, wherein the plate (1b) has a first recess (21), a second recess (22), and a third recess, the second recess (22) and the third recess (23) are filled with a biological cell processing solution, and the openings of the second recess (22) and the third recess are sealed by a sealing sheet (4b), (4c) which is removable.

9. The plate (1b) for biological cell processing operation according to claim 8, wherein at least one of the three biological cell processing solutions filled in the first recess (21), the second recess (22) and the third recess (23) is different from the other biological cell processing solutions, and wherein the different biological cell processing solutions and the remaining two biological cell processing solutions have colors that distinguish them.

10. A kit (10) for pre-treatment operation of biological cell cryopreservation,
wherein the kit (10) for biological cell freezing pre-treatment operation includes a first step plate (1a) for biological cell processing operation as the plate for biological cell processing operation according to any one of claims 1 to 5, a second step plate (1b) for biological cell processing operation, a lid (3a) for the first step plate (1a) for biological cell processing operation and a lid (3b) for the second step plate (1b) for the biological cell processing operation,
the second step plate (1b) for biological cell processing operation has a base portion (5b), an annular wall portion (6b) extending upward from the base portion (5b), a first recess (21) and a second recess (22) located inside the annular wall portion (6b),
the first recess (21) is filled with a first biological cell processing solution for a second step, the second recess (22) is filled with a second biological cell processing solution for the second step, and the openings of the first recess (21) and the second recess (22) are sealed by sealing sheets (4a), (4b) peelably provided at an opening of the first recess (21) and the second recess (22),
the sealing sheets (4a), (4b) have a sheet body portion (41) for sealing the opening of the first recess (21) or the second recess (22) and a gripping portion (42) for peeling that extends from the sheet body portion (41),
the lid (3b) for the second step plate (1b) for the biological cell processing operation is fitted on the second step plate (1b) for the biological cell processing operation and covers the annular wall portion (6b), the first recess (21) and the second recess (22) and rests on the base portion,
when the lid (3b) for the second step plate (1b) for biological cell processing operation rests on the base portion of the second step plate (1b), the gripping portion (42) for peeling the sealing sheet passes between the annular wall portion (6b) and the lid (3b) for the second step plate (1b) for biological cell processing operation and is located on the upper surface portion of the base portion (5b), and is pushed by the lower edge of the lid (3b) for the second step plate (1b) for biological cell processing operation and is in close proximity to the upper surface of the upper surface portion, and cannot be gripped, and when the lid (3b) for the second step plate (1b) for biological cell processing operation is removed from the second step plate (1b) for biological cell processing operation, the gripping portion (42) for peeling can be gripped by raising up and away from the upper surface of the upper surface portion.

11. The kit for pre-treatment operation of biological cell cryopreservation according to claim 10, wherein the biological cell processing solution for the first step is a treatment solution that does not contain cell membrane-permeable cryoprotectant, the first biological cell processing solution for the second step is a low concentration treatment solution containing the cell membrane-permeable cryoprotectant at a low concentration, and the second biological cell treatment solution for the second step is a high concentration treatment solution containing the cell membrane-permeable cryoprotectant at a high concentration.

## Patentansprüche

1. Platte (1a) für Verarbeitungsvorgänge von biologischen Zellen, die eine Vertiefung (8), eine Verarbeitungslösung für biologische Zellen, die in die Vertiefung (8) eingefüllt ist, und eine Versiegelungsfolie (4) aufweist, die abziehbar an der Öffnung der Vertiefung (8) angebracht ist,
wobei die Platte (1a) für biologische Zellverarbeitungsvorgänge einen abnehmbaren Deckel (3a) aufweist, der die Vertiefung (8) abdeckt,
die Platte (1a) einen Basisabschnitt (5a) und einen ringförmigen Wandabschnitt (6a) aufweist, der sich von dem Basisabschnitt (5a) nach oben erstreckt, und die Vertiefung (8) innerhalb des ringförmigen Wandabschnitts (6a) angeordnet ist,
der Basisabschnitt (5a) einen oberen Oberflächenabschnitt (52a) aufweist, der zwischen dem ringförmigen Wandabschnitt (6a) und einer Außenkante des Basisabschnitts (5a) angeordnet ist,
der Deckel (3a) auf die Platte (1a) aufgesetzt ist und den ringförmigen Wandabschnitt (6a) und die Vertiefung (8) abdeckt und auf dem Basisabschnitt (5a) der Platte (1a) aufliegt,
die Versiegelungsfolie (4) einen Folienkörperabschnitt (41) zum Abdichten der Öffnung der Vertiefung (8) aufweist,
wobei
ein Greifabschnitt (42) zum Abziehen vorgesehen ist, der sich von dem Folienkörperabschnitt (41) erstreckt, und
der Deckel (3a) den ringförmigen Wandabschnitt (6a) und die Vertiefung (8) der Platte (1a) abdeckt und auf dem Basisabschnitt (5a) der Platte aufliegt, der Greifabschnitt (42), der zum Abziehen zwischen dem ringförmigen Wandabschnitt (6a) und dem Deckel (3a) verläuft, ist auf dem oberen Oberflächenabschnitt (52a) des Basisabschnitts (5a) angeordnet, wird durch die Unterkante des Deckels (3a) angedrückt und ist in unmittelbarer Nähe zu der Oberseite des Oberflächenabschnitts (52a) angeordnet und kann nicht gegriffen werden, und wenn der Deckel (3a) von der Platte (1a) entfernt wird, kann der Greifabschnitt (42) zum Abziehen durch Hochheben und Wegziehen von der Oberseite des Oberflächenabschnitts (52a) gegriffen werden.

2. Platte (1a) für Verarbeitungsvorgänge von biologischen Zellen nach Anspruch 1, wobei der obere Oberflächenabschnitt (52a) des Basisabschnitts (5a) der Platte einen drückbaren Abschnitt aufweist, der mit einem Finger gedrückt werden kann, wenn ein Abziehvorgang der Versiegelungsfolie (4) unter Verwendung des Greifabschnitts (42) zum Abziehen durchgeführt wird.

3. Platte (1a) für Verarbeitungsvorgänge von biologischen Zellen nach Anspruch 1 oder 2, wobei die Platte (1a) einen flachen Plattenabschnitt (20b) aufweist, der innerhalb des ringförmigen Wandabschnitts (6a) vorgesehen ist, und die Vertiefung (8) in dem flachen Plattenabschnitt (20b) vorgesehen ist.

4. Platte (1a) für Verarbeitungsvorgänge von biologischen Zellen nach Anspruch 3, wobei der flache Plattenabschnitt (20b) innerhalb des ringförmigen Wandabschnitts (6a) und unterhalb einer Oberkante des ringförmigen Wandabschnitts (6a) angeordnet ist.

5. Platte (1a) für Verarbeitungsvorgänge von biologischen Zellen nach Anspruch 4, wobei die Platte (1a) eine ringförmige Rippe aufweist, die an einem Umfang der Vertiefung (8) vorgesehen ist, und einen Flüssigkeitsbehälter, der durch den flachen Plattenabschnitt (20b), die ringförmige Rippe und den ringförmigen Wandabschnitt (6a) gebildet wird, und die Versiegelungsfolie (4) abziehbar an einer Oberseite der ringförmigen Rippe angebracht ist.

6. Platte (1b) für Verarbeitungsvorgänge von biologischen Zellen nach einem der Ansprüche 1 bis 5, wobei die Platte (1b) eine erste Vertiefung (21) und eine zweite Vertiefung (22) aufweist, wobei die zweite Vertiefung (22) mit einer Verarbeitungslösung für biologische Zellen gefüllt ist, wobei eine Öffnung der zweiten Vertiefung (22) durch eine Versiegelungsfolie (4b), welche abziehbar ist, abgedichtet ist.

7. Platte (1b) für Verarbeitungsvorgänge von biologischen Zellen nach Anspruch 6, wobei die in die erste Vertiefung (21) eingefüllte Verarbeitungslösung für biologische Zellen und die in die zweite Vertiefung (22) eingefüllte Verarbeitungslösung für biologische Zellen unterschiedlich sind und wobei eine Verarbeitungslösung für biologische Zellen eine Farbe aufweist, die von der anderen Verarbeitungslösung für biologische Zellen unterscheidbar ist.

8. Platte (1b) für Verarbeitungsvorgänge von biologischen Zellen nach einem der Ansprüche 1 bis 5, wobei die Platte (1b) eine erste Vertiefung (21), eine zweite Vertiefung (22) und eine dritte Vertiefung aufweist, wobei die zweite Vertiefung (22) und die dritte Vertiefung (23) mit einer Verarbeitungslösung für biologische Zellen gefüllt sind und die Öffnungen der zweiten Vertiefung (22) und der dritten Vertiefung durch eine Versiegelungsfolie (4b), (4c), die abnehmbar ist, versiegelt sind.

9. Platte (1b) für Verarbeitungsvorgänge von biologischen Zellen nach Anspruch 8, wobei mindestens eine der drei Verarbeitungslösungen für biologische Zellen, die in die erste Vertiefung (21), die zweite Vertiefung (22) und die dritte Vertiefung (23) gefüllt sind, sich von den anderen Verarbeitungslösungen für biologische Zellen unterscheidet und wobei die unterschiedlichen Verarbeitungslösungen für biologische Zellen und die beiden übrigen Verarbeitungslösungen für biologische Zellen Farben aufweisen, die sie voneinander unterscheiden.

10. Kit (10) für das Vorbehandlungsverfahren biologischer Zellen zur Kryokonservierung, wobei das Kit (10) für das Vorbehandlungsverfahren biologischer Zellen zum Einfrieren eine Platte (1a) für den ersten Schritt der Verarbeitungsvorgänge von biologischen Zellen als Platte für die Verarbeitungsvorgänge von biologischen Zellen nach einem der Ansprüche 1 bis 5, eine Platte (1b) für den zweiten Schritt der Verarbeitungsvorgänge von biologischen Zellen, einen Deckel (3a) für die Platte (1a) für den ersten Schritt der Verarbeitungsvorgänge von biologischen Zellen und einen Deckel (3b) für die Platte (1b) für den zweiten Schritt der Verarbeitungsvorgänge von biologischen Zellen umfasst,
die Platte (1b) für den zweiten Schritt der Verarbeitungsvorgänge von biologischen Zellen einen Basisabschnitt (5b), einen ringförmigen Wandabschnitt (6b), der sich von dem Basisabschnitt (5b) nach oben erstreckt, eine erste Vertiefung (21) und eine zweite Vertiefung (22) aufweist, die innerhalb des ringförmigen Wandabschnitts (6b) angeordnet ist,
die erste Vertiefung (21) mit einer ersten Verarbeitungslösung für biologische Zellen für einen zweiten Schritt gefüllt ist, die zweite Vertiefung (22) mit einer zweiten Verarbeitungslösung für biologische Zellen für den zweiten Schritt gefüllt ist und die Öffnungen der ersten Vertiefung (21) und der zweiten Vertiefung (22) durch Versiegelungsfolien (4a), (4b) versiegelt sind, die abziehbar an einer Öffnung der ersten Vertiefung (21) und der zweiten Vertiefung (22) vorgesehen sind,
die Versiegelungsfolien (4a), (4b) einen Folienkörperabschnitt (41) zum Verschließen der Öffnung der ersten Vertiefung (21) oder der zweiten Vertiefung (22) und einen Greifabschnitt (42) zum Abziehen aufweisen, der sich von dem Folienkörperabschnitt (41) erstreckt,
der Deckel (3b) für die Platte (1b) für den zweiten Schritt der Verarbeitungsvorgänge von biologischen Zellen auf die Platte (1b) für den zweiten Schritt der Verarbeitungsvorgänge von biologischen Zellen aufgesetzt ist und den ringförmigen Wandabschnitt (6b), die erste Vertiefung (21) und die zweite Vertiefung (22) abdeckt und auf dem Basisabschnitt aufliegt,
wenn der Deckel (3b) für die Platte (1b) für den zweiten Schritt der Verarbeitungsvorgänge von biologischen Zellen auf dem Basisabschnitt der Platte (1b) für den zweiten Schritt aufliegt, der Greifabschnitt (42) zum Abziehen der Versiegelungsfolie zwischen dem ringförmigen Wandabschnitt (6b) und dem Deckel (3b) für die Platte (1b) für den zweiten Schritt für Verarbeitungsvorgänge von biologischen Zellen verläuft und auf dem oberen Oberflächenabschnitt des Basisabschnitts (5b) angeordnet ist, und durch die Unterkante des Deckels (3b) für die Platte (1b) für den zweiten Schritt für Verarbeitungsvorgänge von biologischen Zellen angedrückt wird und in unmittelbarer Nähe zur Oberseite des oberen Oberflächenabschnitts angeordnet ist, und er kann nicht gegriffen werden, und wenn der Deckel (3b) für die Platte (1b) für den zweiten Schritt für Verarbeitungsvorgänge von biologischen Zellen von der Platte (1b) für den zweiten Schritt für Verarbeitungsvorgänge von biologischen Zellen entfernt wird, kann der Greifabschnitt (42) zum Abziehen durch Hochheben und Wegziehen von der Oberseite des oberen Oberflächenabschnitts gegriffen werden.

11. Kit für das Vorbehandlungsverfahren der Kryokonservierung biologischer Zellen nach Anspruch 10, wobei die Verarbeitungslösung für biologische Zellen für den ersten Schritt eine Behandlungslösung ist, die kein zellmembrangängiges Gefrierschutzmittel enthält, die erste Verarbeitungslösung für biologische Zellen für den zweiten Schritt eine Behandlungslösung mit niedriger Konzentration ist, die das zellmembrangängige Gefrierschutzmittel in einer niedrigen Konzentration enthält, und die zweite Behandlungslösung für biologische Zellen für den zweiten Schritt eine hochkonzentrierte Behandlungslösung ist, die das zellmembrangängige Gefrierschutzmittel in einer hohen Konzentration enthält.

## Revendications

1. Plateau (1a) pour l'opération de traitement de cellules biologiques ayant un retrait (8), une solution de traitement de cellules biologiques remplie dans le retrait (8) et une feuille de scellement (4) fournie de manière pelable à l'ouverture du retrait (8),
le plateau (1a) pour l'opération de traitement de cellules biologiques ayant un couvercle enlevable (3a) recouvrant le retrait (8),
le plateau (1a) a une portion de base (5a) et une portion de paroi annulaire (6a) s'étendant vers le haut de la portion de base (5a) et le retrait (8) est situé à l'intérieur de la portion de paroi annulaire (6a),
la portion de base (5a) a une portion de surface supérieure (52a) située entre la portion de paroi annulaire (6a) et un bord externe de la portion de base (5a),
le couvercle (3a) est ajusté sur le plateau (1a) et recouvre la portion de paroi annulaire (6a) et le retrait (8) et repose sur la portion de base (5a) du plateau (1a),
la feuille de scellement (4) a une portion de corps de feuille (41) pour sceller l'ouverture du retrait (8),
une portion de mise en prise (42) étant fournie pour le pelage qui s'étend de la portion de corps de feuille (41) et
le couvercle (3a) recouvre la portion de paroi annulaire (6a) et le retrait (8) du plateau (1a) et repose sur la portion de base (5a) du plateau, la portion de mise en prise (42) pour le pelage passe entre la potion de paroi annulaire (6a) et le couvercle (3a), est située sur la portion de surface supérieure (52a) de la portion de base (5a) et est poussée pat le bord inférieur du couvercle (3a) et est proche de la surface supérieure de la portion de surface supérieure (52a) et ne peut pas être mise en prise et quand le couvercle (3a) est enlevé du plateau (1a), la portion de mise en prise (42) pour le pelage peut être mise en prise en montant et à l'écart de la surface supérieure de la portion de surface supérieure (52a).

2. Plateau (1a) pour l'opération de traitement de cellules biologiques selon la revendication 1, dans lequel la portion de surface supérieure (52a) de la portion de base (5a) du plateau a une portion pressable qui peut être pressée par un doigt quand on effectue un travail de pelage de la feuille de scellement (4) en utilisant la portion de mise en prise (42) pour le pelage.

3. Plateau (1a) pour l'opération de traitement biologique selon la revendication 1 ou 2, dans lequel le plateau (1a) a une portion de plateau plate (20b) fournie à l'intérieur de la portion de paroi annulaire (6a) et le retrait (8) est fourni dans la portion de plateau plate (20b).

4. Plateau (1a) pour l'opération de traitement de cellules biologiques selon la revendication 3, dans lequel la portion de plateau plate (20b) est située à l'intérieur de la portion de paroi annulaire (6a) et au-dessous d'un bord de partie supérieure de la portion de paroi annulaire (6a).

5. Plateau (1a) pour l'opération de traitement de cellules biologiques selon la revendication 4, dans lequel le plateau (1a) a une nervure annulaire fournie à une périphérie du retrait (8) et un réservoir de liquide formé par la portion de plateau plate (20b), la nervure annulaire et la portion de paroi annulaire (6a) et la feuille de scellement (4) est collée de manière pelable à une surface supérieure de la nervure annulaire.

6. Plateau (1b) pour l'opération de traitement de cellules biologiques selon l'une quelconque des revendication 1 à 5, dans lequel le plateau (1b) a un premier retrait (21) et un second retrait (22), le second retrait (22) étant rempli avec une solution de traitement de cellules biologiques, une ouverture du second retrait (22) étant scellée par une feuille de scellement (4b) qui est pelable.

7. Plateau (1b) pour l'opération de traitement de cellules biologiques selon la revendication 6, dans lequel la solution de traitement de cellules biologiques remplie dans le premier retrait (21) et la solution de traitement de cellules biologiques remplie dans le retrait (22) sont différentes et une solution de traitement de cellule biologiques ayant une couleur qui est distinguable de l'autre solution de traitement de cellules biologiques.

8. Plateau (1b) pour l'opération de traitement de cellules biologiques selon l'une quelconque des revendication 1 à 5, dans lequel le plateau (1b) a un premier retrait (21), un deuxième retrait (22) et un troisième retrait, le deuxième retrait (22) et le troisième retrait (23) sont remplis avec une solution de traitement de cellules biologiques et les ouvertures du deuxième retrait (22) et du troisième retrait (23) sont scellées par une feuille de scellement (4b), (4c) qui est enlevable.

9. Plateau (1b) pour l'opération de traitement de cellules biologiques selon la revendication 8, dans lequel au moins une des trois solutions de traitement de cellules biologiques remplie dans le premier retrait (21, le deuxième retrait (22) et le troisième retrait (23) est différente des autres solutions de traitement de cellules biologiques et les différentes solutions de traitement de cellules biologiques et les deux solutions de traitement de cellules biologiques restantes ayant des couleurs qui les distinguent.

10. Kit (10) pour l'opération de pré-traitement de cryoconservation de cellules biologiques
dans lequel le kit (10) pour l'opération de pré-traitement de congélation de cellules biologiques comprend un premier plateau d'étape (1a) pour l'opération de traitement de cellules biologiques comme le plateau pour l'opération de traitement de cellules biologiques selon l'une quelconque des revendications 1 à 5, un second plateau d'étape (1b) pour l'opération de traitement de cellules biologiques, un couvercle (3a) pour le premier plateau d'étape (1a) pour l'opération de traitement de cellules biologiques et un couvercle (3b) pour le second plateau d'étape (1b) pour l'opération de traitement de cellules biologiques,
le second plateau d'étape (1b) pour l'opération de traitement de cellules biologiques a une portion de base (5b), une portion de paroi annulaire (6b) s'étendant vers le haut de la portion de base (5b), un premier retrait (21) et un second retrait (22) situés à l'intérieur de la portion de paroi annulaire (6b),
le premier retrait (21) est rempli avec une première solution de traitement de cellules biologiques pour une seconde étape, le second retrait (22) est rempli avec une seconde solution de traitement de cellules biologiques pour la seconde étape et les ouvertures du premier retrait (21) et du second retrait (22) sont scellées par des feuilles de scellement (4a) (4b) fournies de manière pelable à une ouverture du premier retrait (21) et du second retrait (22),
les feuiles de scellement (4a), (4b) ont une portion de corps de feuille (41) pour sceller l'ouverture du premier retrait (21) ou du second retrait (22) et une portion de mise en prise (42) pour le pelage qui s'étend de la première portion de corps de feuille (41),
le couvercle (3b) pour le second plateau d'étape (1b) pour l'opération de traitement de cellules biologiques est ajusté sur le second plateau d'étape (1b) pour l'opération de traitement de cellules biologiques et recouvre la portion de paroi annulaire (6b), le premier retrait (21) et le second retrait (22) et repose sur la portion de base,
quand le couvercle (3b) pour le second plateau d'étape (1b) pour l'opération de traitement de cellules biologiques repose sur la portion de base du second plateau d'étape (1b), la portion de mise en prise (42) pour peler la feuille de scellement passe entre la paroi de portion annulaire (6b) et le couvercle (3b) pour le second plateau d'étape (1b) pour l'opération de traitement de cellules biologiques et est située sur la portion de surface supérieure de la portion de base (5b) et est poussée par le bord inférieur du couvercle (3b) pour le second plateau d'étape (1b) pour l'opération de traitement de cellules biologiques et est proche de la surface supérieure de la portion de surface supérieure et ne peut pas être mise en prise et quand le second couvercle (3b) pour le second plateau d'étape (1b) pour l'opération de traitement de cellules biologiques est enlevé du second plateau d'étape (1b) pour l'opération de traitement de cellules biologiques, la portion de mise en prise (42) pour le pelage peut être mise en prise en montant et à l'écart de la surface supérieure de la portion de surface supérieure.

11. Kit pour l'opération de pré-traitement de cryoconservation de cellules biologiques selon la revendication 10, dans lequel la solution de traitement de cellules biologiques pour la première étape est une solution de traitement qui ne contient pas d'agent cryoprotecteur perméable à la membrane cellulaire, la première solution de traitement de cellules biologiques pour la seconde étape est une solution de traitement à faible concentration contenant l'agent cryoprotecteur perméable à la membrane cellulaire à une faible concentration et la seconde solution de traitement de cellules biologiques pour la seconde étape est une solution de traitement à haute concentration contenant l'agent cryoprotecteur perméable à la membrane cellulaire à une haute concentration.
